# EUROPEAN PATENT SPECIFICATION

(11) **EP 1 487 452 B1**
(45) Date of publication and mention of the grant of the patent: **24.05.2006**
(21) Application number: 03742638.4
(22) Date of filing: 17.02.2003
(51) Int. Cl.: A61K 31/506, A61P 35/00

(54) **USE OF 4-(4-METHYLPIPERAZIN-1-YLMETHYL)-N 4-METHYL-3-(4-PYRIDIN-3-YL) PYRIMIDIN-2-YLAMINO) PHENYL -BENZAMIDE FOR TREATING SEMINOMAS**
VERWENDUNG VON 4-(4-METHYLPIPERAZIN-1-YLMETHYL)-N 4-METHYL-3-(4-PYRIDIN-3-YL) PYRIMIDIN-2-YLAMINO) PHENYL BENZAMID ZUR BEHANDLUNG VON SEMINOMEN
UTILISATION DE 4-(4-METHYLPIPERAZINE-1-YLMETHYL)-N 4-METHYL-3-(4-PYRIDINE-3-YL)PYRIMIDINE-2-YLAMINO)PHENYL|-BENZAMIDE DANS LE TRAITEMENT DE SEMINOMES

(30) Priority: 22.02.2002 US 359049 P
(43) Date of publication of application: 22.12.2004
(73) Proprietor: The Government of the United States of America, as represented by The Department of Veterans Affairs, Washington, DC 20420 (US); Oregon Health & Science University, Portland, OR 97201-4753 (US)
(72) Inventor: HEINRICH, Michael, C., Lake Oswego, OR 97035 (US); CORLESS, Christopher, Portland, OR 97239 (US); KEMMER, Kathleen, Portland, OR 97214 (US)
(74) Representative: de Weerd, Petrus G.W.
(86) International application number: PCT/IB2003/000622
(87) International publication number: WO 2003/070248

(56) References cited:
- WO-A-99/03854
- JOENSUU H ET AL: "TYROSINE KINASE INHIBITOR IMATINIB (STI571) AS AN ANTICANCER AGENT FOR SOLID TUMOURS" ANNALS OF MEDICINE, FINNISH MEDICAL SOCIETY DUODECIM, HELSINKI, FI, vol. 33, no. 7, 2001, pages 451-455, XP001131999 ISSN: 0785-3890
- SMITHEY, B. E. ET AL.: "c-kit espression in pediatric solid tumors" THE AMERICAN JOURNAL OF SURGICAL PATHOLOGY, vol. 26, no. 4, April 2002 (2002-04), pages 486-492, XP009008983
- TIAN, Q. ET AL.: "Activating c-kit gene mutations in human germ cell tumors" AMERICAN JOURNAL OF PATHOLOGY, vol. 154, no. 6, June 1999 (1999-06), pages 1643-1647, XP002237659

## Description

The invention relates to the use of 4-(4-methylpiperazin-1-ylmethyl)-N-[4-methyl-3-(4-(pyridin-3-yl)pyrimidin-2-ylamino)phenyl]-benzamide (hereinafter: "COMPOUND I") or a pharmaceutically acceptable salt thereof for the manufacture of pharmaceutical compositions for the treatment of seminomas.

Testicular germ cell tumors are uncommon neoplasms, accounting for only 1 % to 2% of malignancies in North American males. They are, however, the commonest malignancies in men aged 20-34 and epidemiological studies have shown a doubling of the incidence rate in the past 30 years. Approximately 45% of germ cell tumors are seminomas and the majority of patients will present with clinical stage I disease. Only 15% to 20% of patients have infradiaphragmatic lymph node Involvement (stage II disease) and less than 5% present with distant metastatic disease.

Standard treatment for stage I seminomas, in which the tumor is confined to the testis and a lymphangiogram is negative, involves orchidectomy followed by ipsilateral irradiation of the para-aortic and pelvic lymph nodes. The treatment for Stage II seminomas, in which the tumor has spread to lymph nodes below the diaphragm, involves radiation treatment that extends to the involved anatomy.
About 10-20% of seminomas patients harbor micrometastases in the draining lymph nodes. Marks et al., J. Urol. 143:524 (1990). Therefore, the irradiation of regional lymph nodes as part of the standard treatment of Stage I seminomas is unnecessary in approximately 85% of all seminoma patients. Although post-orchidectomy radiation is generally well tolerated, local complications have been reported to include a higher incidence of second-site malignancies, impaired fertility, and persistent scrotal edema. Hunter, et al., Cancer 64:1608 (1989); Thomas, et al., J. Urol. 12:313 (1989).

The instant invention is a response to the need for an alternative therapy in the treatment of seminomas, especially to decrease the need for subsequent radiation or chemotherapy treatment and reduce consequent infertility.

It has now surprisingly been demonstrated that seminomas can be successfully treated with COMPOUND I, or pharmaceutically acceptable salt thereof.

The present invention thus concerns the use of 4-(4-methylpiperazin-1-ylmethyl)-N-[4-methyl-3-(4-(pyridin-3-yl)pyrimidin-2-ylamino)phenyl]-benzamide having the formula I 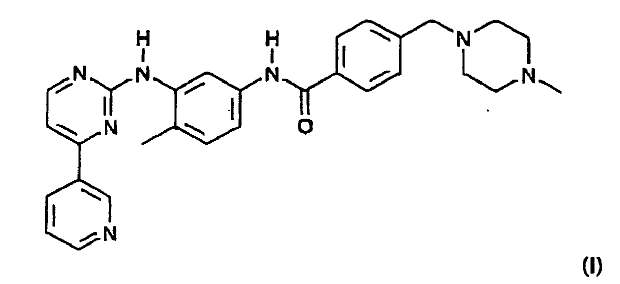 or a pharmaceutically acceptable salt thereof, for the manufacture of a medicament for treating seminomas.

The present invention particularly concerns the use of COMPOUND I for the manufacture of a medicament for treating seminomas associated with c-Kit mutations sensitive to COMPOUND I.

The present invention most particularly concerns the use of COMPOUND I for the manufacture of a medicament for treating seminomas associated with YB23D, N822K or D816H mutations in the c- Kit tyrosine kinase.

4-(4-methylpiperazin-1-ylmethyl)-N-[4-methyl-3-(4-(pyridin-3-yl)pyrimidin-2-ylamino)phenyl]-benzamide or a pharmaceutically acceptable salt crystal form thereof will be referred herein as COMPOUND I.

The term ''4-(4-methylpiperazin-1-ylmethyl)-N-[4-methyl-3-(4-(pyridin-3-yl)pyrimidin-2-yl-amino)phenyl]-benzamide" includes the β-crystal form as described in the European patent application No. 998 473.

The preparation of COMPOUND I and the use thereof, especially as an anti-tumour agent, are described in Example 21 of European patent application EP-A-0 564 409, which was published on 6 October 1993, and in equivalent applications and patents in numerous other countries, e.g. in US patent 5,521,184 and in Japanese patent 2706682.

By the term "seminomas associated with COMPOUND I-sensitive Kit mutations" the applicant means a seminoma disease in which the germ cell tumors contain c-Kit having one or more mutations, the c-Kit mutant being sensitive to inhibition of its tyrosine kinase activity by COMPOUND I with an *in vitro* IC₅₀ of approximately 10 nM to 50µM preferably 10 nM to 10µM most preferably10 nM to 1µM.

By the term "seminomas associated with Y823D, N822K and/or D816H mutations in the c-Kit tyrosine kinase" the applicant means a seminoma disease in which the germ cell tumors contain human c-Kit tyrosine kinase having one or more of the following mutations Y823D, N822K or D816H.

The names of the amino acids are either written out or the one letter or three letter codes are used. Mutations are referred to by accepted nomenclature, e.g. "Ala380Thr" or "380 Ala→Thr" both indicating that alanine at position 380 is replaced by threonine. The amino acid numbers indicating the position of the mutation refer to the amino acid numbereing of the native human c-Kit protein as given in the SwissProt database under Accession Number P10721.

Y823D indicating that the amino acid tyrosine at position 823 is replaced by aspartate
N822K indicating that the amino acid asparagine at position 822 is replaced by lysine
D816H indicating that the amino acid aspartate at position 816 is replaced by histidine
The term "treatment" as used herein means curative treatment and prophylactic treatment.
The term "curative" as used herein means efficacy in treating ongoing episodes of seminomas.
The term "prophylactic" means the prevention of the onset or recurrence of seminomas.

Pharmaceutically acceptable salts of COMPOUND I are pharmaceutically acceptable acid addition salts, like for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid.

The monomethanesulfonic acid addition salt of COMPOUND I (hereinafter "COMPOUND I mesylate") and a preferred crystal form thereof are described in PCT patent application WO99/03854 published on January 28, 1999.

Depending on species, age, individual condition, mode of administration, and the clinical picture in question, effective doses, for example daily doses of about 100-1000 mg, preferably 200-600 mg, especially 400 mg, are administered to warm-blooded animals of about 70 kg bodyweight. For adult patients with unresectable and/or metastatic malignant seminomas, a starting dose of 400 mg daily can be recommended. For patients with an inadequate response after an assessment of response to therapy with 400 mg daily, dose escalation can be safely considered and patients may be treated as long as they benefit from treatment and in the absence of limiting toxicities.

The invention relates also to the administration to a human subject having seminoma, a COMPOUND I or a pharmaceutically acceptable salt thereof, which comprises administering a pharmaceutically effective amount of COMPOUND I or a pharmaceutically acceptable salt thereof to the human subject. Preferably administered once daily for a period exceeding 3 months. The invention relates especially to such method wherein a daily dose of 200 to 600 mg, especially 400-600 mg, preferably 400 mg, of COMPOUND I mesylate is administered.

It can be shown by established test models and especially those test model described herein that the COMPOUND I or a pharmaceutically acceptable salt thereof, results in a more effective prevention or preferably treatment of seminomas. The person skilled in the pertinent art is fully enabled to select a relevant test model to prove the hereinbefore and hereinafter indicated therapeutic indications and beneficial effects. The pharmacological activity may, for example, be demonstrated in a clinical study or in the test procedure as essentially described hereinafter.

The Kit protooncogene encodes a 145-kd transmembrane tyrosine kinase (TK) closely related to platelet derived growth factor receptor (PDGFR), monocyte-colony stimulating factor receptor (c-fms) and fms-like tyrosine kinase 3 (FLT3). It is expressed on hematopoietic stem cells, mast cells, melanocytes, interstitial cells of Cajal, intraepithelial lymphocytes, and germ cells. Studies of naturally occurring murine models of Kit deficiency have demonstrated that Kit is required in utero for the survival of germ cells. Postnatally, Kit is expressed by Leydig cells, spermatogonia and round spermatids. Seminomas and intratubular germ cell neoplasias (ITGCN) almost always express membrane associated Kit (as demonstrated by immunohistochemistry). In contrast, only a minority of non-seminoma germ cell tumors (GCT) express cytoplasmic, but not membranous, Kit. In mixed GCTs, membranous Kit expression is limited to the seminoma component.
In one case of mixed ovarian dysgerminoma/yolk sac tumor the mutation was detected in both components of the tumor. However, membranous Kit immunoreactivity was limited to the dysgerminoma component, whereas the yolk sac component had only weak cytoplasmic Kit staining. These results suggest that mutational or autocrine/paracrine Kit activation may play a role in the development of ITGCN and seminomas/dysgerminomas but that down-regulation or loss of normal Kit signaling in these cells is associated with progression to non-seminomatous GCT.
Thus, the applicant decided to study the efficacy of COMPOUND I in inhibiting some constitutively activated Kit kinase isoforms associated with seminoma tumors.

### METHODS

**Analysis of Kit mutations**: Cases of pure seminomas were selected from archival pathology specimens. Sections were prepared from formalin-fixed, paraffin-embedded specimens and trimmed to enrich for tumor cells. In some cases, laser capture microscopy was used to enrich for tumor cells. In a minority of patients, fresh frozen tumor specimens were available and were used to prepare genomic DNA and/or RNA, as described by Rader A, et col. (Cancer 2001; 93:275) and Lux ML, et al. (Am.J.Pathol. 2000; 156:791-795).

PCR of genomic DNA was performed as previously described (Rader A et al.), using the primer pairs listed below (5'-3' notation): Exon 9F ATGCTCTGCTTCTGTACTGCC; Exon 9R AGAGCCTAAACATCCCCTTA; Exon 11 F CCAGAGTGCTCTAATGACTG; Exon 11 R ACCCAAAAAGGTGACATGGA; Exon 13F CATCAGTTTGCCAGTTGTGC; Exon 13R ACACGGCTTTACCTCCAATG; Exon 17F TGTATTCACAGAGACTTGGC; Exon 17R GGATTTACATTATGAAAGTCACAGG.

Amplicons of Kit were analyzed by D-HPLC using a Transgenomic WAVE instrument as previously described by Choy YS, et al. (Annals of Human Genetics 1999; 63:383-391). Denaturing temperatures for detection of point mutations were optimized for each primer pair (Exon 9 58° C, Exon 11 57° C, Exon 13 59° C, Exon 17 58° C). Amplicons were bidirectionally sequenced and the identity of all mutations were confirmed by analysis of a second, independent amplification product (Rader A et al.). In cases with available frozen material, the entire Kit cDNA was sequenced (Lux ML, et al.).

**Reagents**: COMPOUND I mesylate. Fresh 10 mM stock solutions of inhibitor were made before each experiment by dissolving compound in 1 ml Phosphate-Buffered Saline (PBS; Gibco-BRL).

**Antibodies:** A polyclonal rabbit anti-Kit antibody (c- Kit Ab-1) was used at a dilution of 1:500 (c-Kit Ab-1; Oncogene, Cambridge, MA). An anti-phosphotyrosine antibody (PY20) was used at a dilution of 1:1000 (PY20 Transduction Laboratories; Lexington, KY). Peroxidase conjugated goat anti-mouse antibody was used at a dilution of 1:5000 and goat anti-rabbit antibody at a dilution of 1:10,000 (Pierce; Rockford, IL).

**In vitro studies**: Plasmids encoding mutant Kit cDNAs were generated by site directed mutagenesis of the wild-type cDNA. All mutations were confirmed by bi-directional sequencing. The CHO (Chinese hamster ovary) cell line was obtained from American Type Culture Collection (ATCC) and maintained as previously described by Bold G, et al. ([published erratum appears in J Med Chem 2000 Aug 10;43(16):3200]. Journal of Medicinal Chemistry 2000; 43:2310-2323).

Cells were transiently transfected with plasmids encoding cDNAs for wild-type or mutant Kit proteins using Lipofectamine PLUS (Invitrogen Life Technologies) according to the manufacturer's protocol. Twenty-four hours after transfection the cells were treated with control media or media containing various concentrations of COMPOUND I mesylate for 90 minutes. The cells were then harvested and protein lysates prepared and analyzed for Kit activation as described previously (Lux ML, et al.).

### RESULTS

### Example 1: Spectrum and frequency of Kit mutations in pure seminomas

To further investigate the type and frequency of KIT mutations occurring in seminomas, we analyzed forty-three archival cases. All tumors were pure seminomas; in cases with heavy lymphocytic infiltrates laser capture microdissection was performed to enrich for tumor DNA. PCR amplimers were screened by denaturing HPLC (Transgenomic WAVE system) and detected mutations were confirmed by direct DNA sequencing. Some cases contained in-frame point mutations in exon 17, including cases with the D816H mutation. Interestingly, the majority of the mutations were D816V, but examples of N822K and Y823D were also found (see Table for summary). The D816V, N822K , and Y823D mutations have not been previously described in seminomas.

### COMPOUND I mesylate inhibits some constitutively activated Kit kinase isoforms associated with seminomas tumors.

The selectivity of COMPOUND I mesylate is related to its ability to reversibly bind to the ATP-binding pocket of ABL, Kit, and PDGFR but not other tyrosine kinases. While the molecular mechanism(s) by which mutations of Kit lead to kinase activation are not fully understood, it is possible that some mutations could inhibit the interaction of COMPOUND I mesylate with the ATP-binding pocket. To examine this possibility, representative constitutively-activated Kit oncoproteins from seminomas were expressed by transient transfection in CHO cells and examined for sensitivity to inhibition by COMPOUND I mesylate. As shown above, mutant Kit isoforms with amino acid substitutions at residue 822 or 823 were highly sensitive to COMPOUND I mesylate with an *in vitro* IC₅₀ of approximately 100-200 nM. In contrast, the D816V mutation that is commonly associated with human mastocytosis, is completely resistant to COMPOUND I mesylate even using concentrations of 5-10 µM. The kinase activity of the D816H mutant isoform was less inhibited by COMPOUND I mesylate than that of wild type Kit. The *in vitro* IC₅₀ was approximately 1 µM.

**DATA SUMMARY**

| Mutation | D816V | D816H | N822K | Y823D |
|---|---|---|---|---|
| COMPOUND I | No | Yes | Yes | Yes |
| Sensitive | | | | |

Taken together, these results suggest that COMPOUND I has an unexpected potential for the treatment of seminomas. The efficacy of COMPOUND I in seminomas is tested by clinical trials in advanced metastatic disease refractory to chemotherapy or in a "window of opportunity" neoadjuvant setting in patients with low volume retroperitoneal disease prior to their radiotherapy.
Future development could include use of COMPOUND I as an adjuvant therapy in patients with Stage I disease in an attempt to decrease the need for subsequent radiation or chemotherapy treatment and perhaps reduce consequent infertility.

### Very good tolerability of COMPOUND I mesylate treatment.

Treatment with COMPOUND I mesylate was well tolerated overall. No hair loss was observed, and the patient reported only mild occasional nausea related to swallowing of the drug capsules, lasting for about 15 minutes improved after taking drug with food. Blood cell count changes were unremarkable. Her blood haemoglobin level varied between 118 g/L and 125 g/L during COMPOUND I mesylate therapy (the pretreatment value was 120 g/L), the white blood cell count from 3.2 to 4.4 x 10⁹/L (5.5 x 10⁹/L), the granulocyte count from 1.52 to 2.39 x 10⁹/L (3.2 x 10⁹/L), and the platelet count from 261 to 365 x 10⁹/L (360 x 10⁹/L). No drug-related liver, renal or cardiac toxicity was observed. The main subjective toxicity [all Grade 1 (NCl CTC version 2.0)] consisted of increased frequency of bowel movements (2 to 4 times a day), occasional muscle cramps in the legs, slight transient ankle oedema, and a Herpes zoster infection with rash located on the left ventral (LV) dermatome was diagnosed during COMPOUND I mesylate therapy. The World Health Organization (WHO) performance status improved from 1 (cancer related symptoms present) to 0 (normal) during COMPOUND I mesylate therapy.

### Example 2: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form.

Capsules containing 119.5 mg of the compound named in the title (=COMPOUND I mesylate) corresponding to 100 mg of COMPOUND I (free base) as active substance are prepared in the following composition:

### Composition

| | |
|---|---|
| COMPOUND I mesylate | 119.5 mg |
| Cellulose MK GR | 92 mg |
| Crospovidone XL | 15 mg |
| Aerosil 200 | 2 mg |
| Magnesium stearate | 1.5 mg |
| | 230 mg |

The capsules are prepared by mixing the components and filling the mixture into hard gelatin capsules, size 1.

### Example 3: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form.

Capsules containing 119 mg of the compound named in the title (=COMPOUND I mesylate) as active substance are prepared in the following composition:

### Composition

| | |
|---|---|
| Active substance | 119 mg |
| Avicel | 200mg |
| PVPPXL | 15 mg |
| Aerosil | 2 mg |
| Magnesium stearate | 1.5 mg |
| | 337.5 mg |

The capsules are prepared by mixing the components and filling the mixture into hard gelatin capsules, size 1.

## Claims

1. The use of 4-(4-methylpiperazin-1-ylmethyl)-N-[4-methyl-3-(4-(pyridin-3-yl)pyrimidin-2-yl-amino)phenyl]-benzamide of the formula I 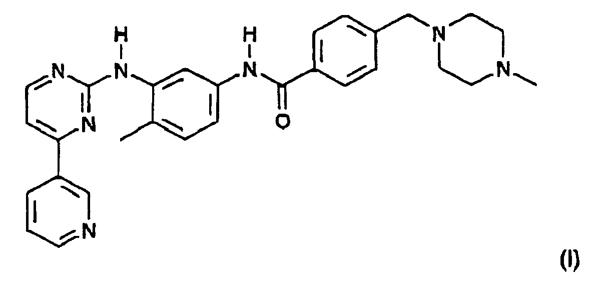 or a pharmaceutically acceptable salt thereof for the manufacture of pharmaceutical compositions for use in the treatment of seminomas.

2. Use according to claim 1, wherein seminoma is associated with c-Kit mutations sensitive to COMPOUND I.

3. Use according to claim 1, wherein seminoma is associated with Y823D, N822K or D816H mutations in the c- Kit tyrosine kinase.

4. Use according to claim 1 wherein a pharmaceutically acceptable acid addition salt of 4-(4-methylpiperazin-1-ylmethyl)-N-[4-methyl-3-(4-(pyridin-3-yl)pyrimidin-2-ylamino)phenyl]-benzamide of the formula I is used.

5. Use according to claim 1, wherein the pharmaceutically acceptable salt is monomethanesulfonate salt.

## Patentansprüche

1. Verwendung von 4-(4-Methylpiperazin-1-ylmethyl)-N-[4-methyl-3-(4-(pyridin-3-yl)pyrimidin-2-ylamino)-phenyl]benzamid der Formel I 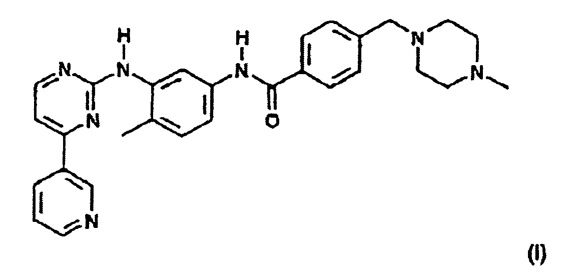 oder eines pharmazeutisch annehmbaren Salzes hiervon zur Herstellung von pharmazeutischen Zusammensetzungen zur Behandlung von Seminomen.

2. Verwendung nach Anspruch 1, worin das Seminom mit c-Kit Mutationen assoziiert ist, die gegenüber Verbindung I empfindlich sind.

3. Verwendung nach Anspruch 1, worin das Seminom mit Y823D, N822K oder D816H Mutationen in der c-Kit Tyrosinkinase assoziiert ist.

4. Verwendung nach Anspruch 1, worin ein pharmazeutisch annehmbares Säureadditionssalz von 4-(4-Methylpiperazin-1-ylmethyl)-N-[4-methyl-3-(4-pyridin-3-yl)pyrimidin-2-ylamino)phenyl]benzamid der Formel I verwendet wird.

5. Verwendung nach Anspruch 1, worin das pharmazeutisch annehmbare Salz das Monomethansulfonatsalz ist.

## Revendications

1. Utilisation de 4-(4-méthylpipérazin-1-ylméthyl)-N-[4-méthyl-3-(4-(pyridin-3-yl)pyrimidin-2-yl-amino)phényl]-benzamide de formule I 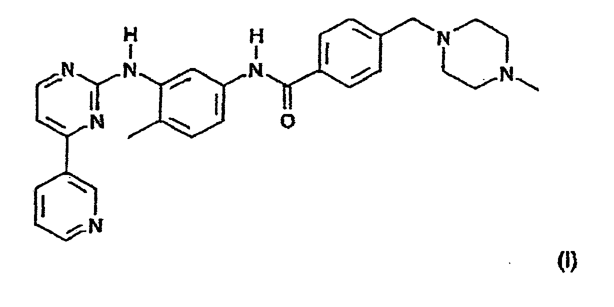 ou un de ses sels pharmaceutiquement acceptables pour la fabrication de compositions pharmaceutiques utilisables dans le traitement de séminomes.

2. Utilisation selon la revendication 1, **caractérisée en ce que** le séminome est associé à des mutations c-Kit sensibles au COMPOSE I.

3. Utilisation selon la revendication 1, **caractérisée en ce que** le séminome est associé aux mutations Y823D, N822K ou D816H dans la tyrosine kinase c-Kit.

4. Utilisation selon la revendication 1, **caractérisée en ce qu'**un sel d'addition d'acide pharmaceutiquement acceptable de 4-(4-méthylpipérazin-1-ylméthyl)-N-[4-méthyl-3-(4-(pyridin-3-yl)pyrimidin-2-yl-amino)phényl]-benzamide de formule I est employé.

5. Utilisation selon la revendication 1, **caractérisée en ce que** le sel pharmaceutiquement acceptable est un sel de monométhanesulfonate.
